(19) Europäisches Patentamt
European Patent Office
Office européen des brevets

(11) **EP 3 469 926 A1**

(12) **EUROPEAN PATENT APPLICATION**
published in accordance with Art. 153(4) EPC

(43) Date of publication:
**17.04.2019 Bulletin 2019/16**

(51) Int Cl.:
**A24F 47/00** (2006.01)   **A61M 15/06** (2006.01)

(21) Application number: **16907222.0**

(22) Date of filing: **27.06.2016**

(86) International application number:
**PCT/JP2016/069012**

(87) International publication number:
**WO 2018/002989 (04.01.2018 Gazette 2018/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Japan Tobacco, Inc.**
**Tokyo 105-8422 (JP)**

(72) Inventors:
• **NAKANO, Takuma**
**Tokyo 130-8603 (JP)**
• **MATSUMOTO, Hirofumi**
**Tokyo 130-8603 (JP)**
• **YAMADA, Manabu**
**Tokyo 130-8603 (JP)**

(74) Representative: **Isarpatent**
**Patent- und Rechtsanwälte Behnisch Barth Charles**
**Hassa Peckmann & Partner mbB**
**Friedrichstrasse 31**
**80801 München (DE)**

(54) **FLAVOR INHALER CARTRIDGE AND FLAVOR INHALER HAVING FLAVOR INHALER CARTRIDGE**

(57)     Provided is a flavor inhaler cartridge that comprises: a liquid storage part that stores an aerosol-generating liquid; and a porous heating body that is provided with a positive electrode and a negative electrode and that, when current has been passed between the positive electrode and the negative electrode, emits heat and thereby atomizes aerosol-generating liquid supplied from the liquid storage part. The relative standard deviation of the resistance value of the porous heating body as measured under prescribed experimental conditions is no more than 5.0%.

Fig.1

**Description**

[Technical Field]

The present invention relates to a flavor inhaler cartridge and a flavor inhaler having the flavor inhaler cartridge.

[Background Art]

Flavor inhalers designed to enable a user to inhale an aerosol formed by vaporizing a liquid containing a flavor component and a fragrance using electrical energy are known.

Patent Document 1 describes a planar composite as a heating element for performing atomization of a liquid stored in a flavor inhaler. Due to its structure, the planar composite described in Patent Document 1 also contributes to capillary transport of the liquid and is also used to suction the liquid. In addition, according to the invention described in Patent Document 1, due to the heating element being a planar composite, atomization can be performed at high specific evaporation capacity and at high evaporator efficiency. Patent Document 1 describes a relationship between porosity of the planar composite and evaporator efficiency and indicates that, the higher the porosity of the planar composite serving as a heating element, the larger a portion of heat generated by the planar composite is used for the atomization of a liquid absorbed in pores of the planar composite.

The invention described in Patent Document 1 focuses on increasing the porosity of the planar composite in order to achieve high evaporator efficiency and, to achieve this, adopts structures such as a woven structure, an open-pore fiber structure, an open-pore sintered structure, an open-pore foam, and an open-pore deposition structure as a structure of the planar composite.

[Citation List]

[Patent Document]

[Patent Document 1] Japanese Patent No. 5612585

[Summary of Invention]

[Technical Problem]

In the invention described in Patent Document 1, suction and atomization of a liquid is performed using a material referred to as a planar composite.

While it is described that an atomization rate of the planar composite is increased by increasing the porosity thereof, a relationship between the structure of the planar composite and uniform heat generation over the entire planar composite is not described.

It has been found that, when using a material with high porosity such as that described in Patent Document 1 as an element for suctioning and heating a liquid containing a flavor source when atomizing the liquid during the use of a flavor inhaler, localized heat generation may occur in the material. The occurrence of localized heat generation in the material as an element for suctioning and heating the liquid results in hastening deterioration of the material and impairing a function thereof and, at the same time, may cause a variation in atomization amounts of the liquid.

In consideration of the above, an object of the present invention is to provide a flavor inhaler cartridge and a flavor inhaler which include a porous heating element in which uniform heat generation occurs over the entire heating element as a heating element for suctioning and atomizing a liquid including a flavor source.

[Solution to Problem]

The present inventors accomplished the present invention based on their discovery that the problem described above can be solved with a flavor inhaler cartridge including: a liquid storage portion which stores an aerosol-forming liquid; and a porous heating element which is provided with a positive electrode and a negative electrode and which atomizes the aerosol-forming liquid supplied from the liquid storage portion by generating heat when a current is passed between the positive electrode and the negative electrode, wherein a relative standard deviation of resistance values of the porous heating element as measured under specific test conditions is 5.0% or less.

Specifically, the present invention provides the following.

[1] A flavor inhaler cartridge, including:

a liquid storage portion which stores an aerosol-forming liquid; and

a porous heating element which is provided with a positive electrode and a negative electrode and which atomizes the aerosol-forming liquid supplied from the liquid storage portion by generating heat when a current is passed between the positive electrode and the negative electrode, wherein

a relative standard deviation of resistance values of the porous heating element as measured under the following test conditions is 5.0% or less

test conditions: the positive electrode and the negative electrode are arranged on the porous heating element so as to create an 8 mm-long electrical path, and a resistance value is measured a total of 30 times, the measurements of the resistance value being performed by changing a position of the positive electrode or the negative electrode for each measurement so as to maintain the 8 mm length of the electrical path.

[2] The flavor inhaler cartridge according to [1], wherein a nominal pore diameter of the porous heating element is 1000 $\mu$m or less.

[3] The flavor inhaler cartridge according to [1], wherein a porosity of the porous heating element is 50% or more.

[4] The flavor inhaler cartridge according to any one of [1] to [3], wherein the porous heating element is fabricated by a manufacturing method including: a step of applying a conductivity-imparting process to a porous resin; a first plating step of applying metal plating to the porous resin having been subjected to the conductivity-imparting process; and a heat treatment step of removing the porous resin.

[5] The flavor inhaler cartridge according to [4], wherein the metal used in the first plating step is nickel or chromium.

[6] The flavor inhaler cartridge according to [4] or [5], including, after the heat treatment step, a second plating step of applying plating with a metal that differs from the metal used in the first plating step.

[7] The flavor inhaler cartridge according to any one of [1] to [3], wherein the porous heating element includes:

a step of retaining a slurry obtained by mixing a carbon source with silicon powder on a skeleton of a porous structure and subsequently drying the slurry;

a first heating step of heating the dried porous structure under vacuum or under an inert atmosphere to obtain a porous composite;

a second heating step of heating, under vacuum or under an inert atmosphere, the porous composite obtained in the first heating step to obtain a porous sintered body; and

a third heating step of heating the obtained porous sintered body under vacuum or under an inert atmosphere to obtain the porous heating element.

[8] The flavor inhaler cartridge according to [7], wherein the carbon source is a phenolic resin.

[9] A flavor inhaler including the flavor inhaler cartridge according to any one of [1] to [8].

[Effects of Invention]

With the flavor inhaler cartridge according to the present invention, by configuring a porous heating element which is a heating element used for suctioning and atomizing a liquid so as to have the specific resistance values described above, local heat generation of the porous heating element can be prevented.

[Brief Description of Drawings]

Fig. 1 is a schematic view of an electronic cigarette as an example of a flavor inhaler according to a first embodiment.

Fig. 2 is a schematic view of an electronic cigarette as an example of a flavor inhaler according to the first embodiment.

Fig. 3 is a diagram showing a schematic configuration of a cartridge according to the first embodiment.

Fig. 4 is a diagram showing a planer structure of a porous metallic sheet according to an embodiment.

Fig. 5 is a diagram showing a cartridge according to a second embodiment.

Fig. 6 is a diagram showing a cartridge according to a third embodiment.

Fig. 7 is a diagram showing a cartridge according to a fourth embodiment.

Fig. 8 is a diagram showing a cartridge according to a fifth embodiment.

Fig. 9 is a diagram showing a cartridge according to a first modification of the fifth embodiment.

Fig. 10 is a diagram showing a porous heating sheet according to a second modification of the fifth embodiment.

Fig. 11A is a diagram showing an electronic cigarette according to a sixth embodiment.

Fig. 11B is a diagram showing a cartridge according to the sixth embodiment.

Fig. 12A is a diagram showing an electronic cigarette according to a modification of the sixth embodiment.

Fig. 12B is a diagram showing a cartridge according to a modification of the sixth embodiment.

Fig. 13 is a schematic view showing measurement points of a resistance value of a porous heating element included in a flavor inhaler cartridge.

Fig. 14 is a diagram showing a relationship between a relative standard deviation and measurement intervals of resistance values of porous heating elements according to Example 1 and Comparative example 1.

[Description of Embodiments]

Embodiments of a flavor inhaler according to the present invention and a cartridge and a porous heating element applied to the flavor inhaler according to the present invention will now be described with reference to the drawings. It is to be understood that dimensions, materials, shapes, relative arrangements, and the like of components described in the present embodiments are not intended to limit the technical scope of the present invention to the components described in the embodiments unless otherwise noted.

<First embodiment>

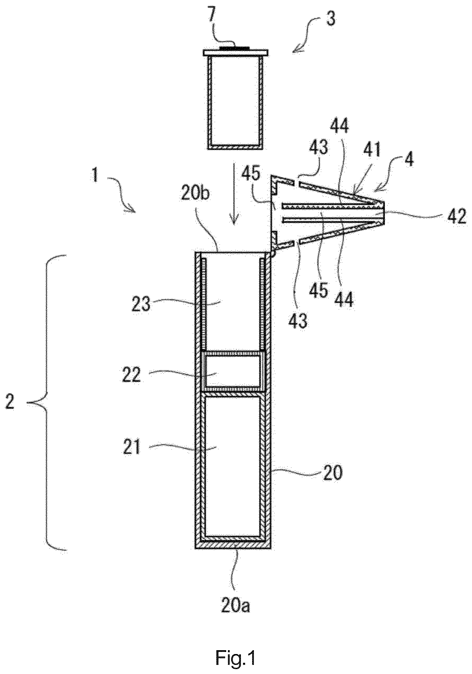

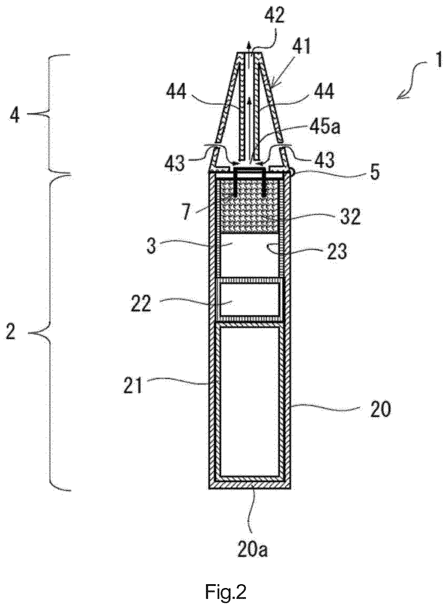

Figs. 1 and 2 are schematic views of an electronic cigarette 1 as an example of an aerosol inhaler (a flavor inhaler) according to a first embodiment. The electronic cigarette 1 includes a main body portion 2 and a mouthpiece portion 4. The main body portion 2 has a main body-side housing 20 in which a battery 21, an electronic control portion 22, and the like are housed. The battery 21 may be a rechargeable battery such as a lithium-ion secondary battery.

The electronic control portion 22 is a computer which controls the entire electronic cigarette 1. The electronic control portion 22 may be a micro-controller which has a circuit board (not shown) mounted with a processor, a memory, and the like.

The main body-side housing 20 is, for example, a bottomed cylindrical shell, and the battery 21 and the electronic control portion 22 are arranged in this order from a side of a bottom surface 20a. In addition, a hollow housing cavity 23 for housing a cartridge 3 is formed on a side of an open end 20b positioned at an upper end of the main body-side housing 20. The cartridge 3 is an assembly which integrates a liquid tank (a liquid storage portion) for housing an aerosol-forming liquid that forms an aerosol when atomized by electrical heating with a porous heating element that heats and atomizes the aerosol-forming liquid, and details thereof will be provided later. In the electronic cigarette 1 according to the present embodiment, the electronic control portion 22 and the battery 21 may be provided in this order from the side of the bottom surface 20a or display means such as an LED or a display may be provided at an arbitrary position of the bottomed cylindrical shell.

The electronic control portion 22 and the battery 21 are connected via electric wiring, and supply of power from the battery 21 to a porous heating element 7 as an atomizing portion of the cartridge 3 is controlled by the electronic control portion 22. In addition, for example, a smoking switch (not shown) to be operated by a user may be provided on the main body-side housing 20. The smoking switch is connected to the electronic control portion 22 via electric wiring, and when the electronic control portion 22 detects that the smoking switch has been operated to turn on the smoking switch, the electronic control portion 22 controls the battery 21 and causes the battery 21 to feed power to a porous heating element of the cartridge 3.

Next, the mouthpiece portion 4 will be described. The mouthpiece portion 4 is connected to the main body portion 2 by a hinge 5. Fig. 1 shows a state where the mouthpiece portion 4 is arranged in an open position to enable the cartridge 3 to be replaced (housed or extracted) with respect to the housing cavity 23 of the main body portion 2. In the state where the mouthpiece portion 4 is arranged in the open position, the housing cavity 23 is opened to the outside.

On the other hand, Fig. 2 shows a state where the mouthpiece portion 4 has been rotated by approximately 90 degrees from the open position and is arranged in a closed position. In the state where the mouthpiece portion 4 is arranged in the closed position, the mouthpiece portion 4 covers, from above, the housing cavity 23 and the cartridge 3 housed in the housing cavity 23. Alternatively, in the electronic cigarette 1 according to the present embodiment, the mouthpiece portion 4 and the main body portion 2 (the battery assembly) may be configured to be attachable/detachable. Engaging means between the mouthpiece portion 4 and the main body portion 2 in this case is not particularly limited and known connecting means including a connection by a screw and a connection such as a fitting connection by a sleeve member can be used.

The mouthpiece portion 4 has a housing 41. The housing 41 of the mouthpiece portion 4 has a shape which is tapered toward a tip side so that the user can readily hold the mouthpiece portion 4 between the user's teeth, and a suction port 42 is formed on a tip side of the housing 41. In addition, an air intake port 43 is provided in the housing 41 of the mouthpiece portion 4. Furthermore, a cylindrical baffle partition 44 which connects to the suction port 42 is provided inside the housing 41 of the mouthpiece portion 4 and an internal passage 45 is formed by the baffle partition 44. The internal passage 45 of the mouthpiece portion 4 is communicated with the suction port 42 and the air intake port 43. When the user smokes, outside air taken into the housing 41 from the outside through the air intake port 43 travels along the internal passage 45 and reaches the suction port 42. In the internal passage 45, an atomization cavity 45a is formed in a vicinity of an upper surface of the cartridge 3. The cartridge 3 forms an aerosol by electrically heating the aerosol-forming liquid stored in the liquid tank to vaporize the aerosol-forming liquid and mixing the vaporized aerosol-forming liquid with air inside the atomization cavity 45a. The formed aerosol is guided to the suction port 42 via the atomization cavity 45a and the internal passage 45, and the user can suction the aerosol through the suction port 42.

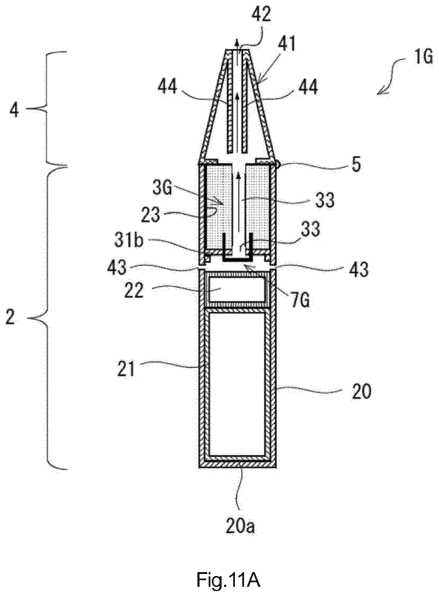

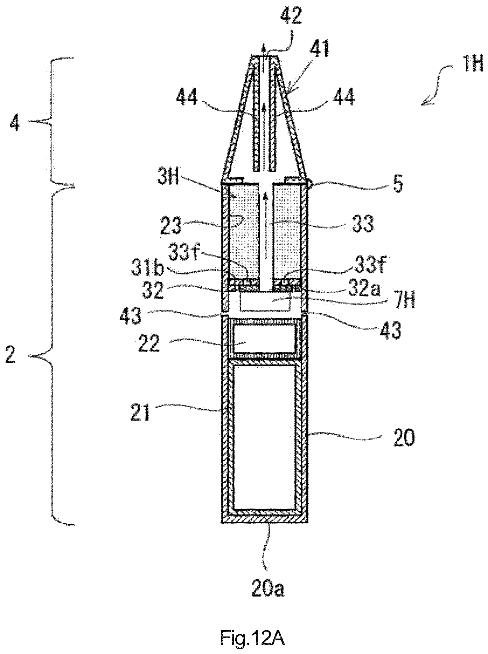

Alternatively, a suction sensor (not shown) may be installed in place of the smoking switch in the main body-side housing 20 and the electronic cigarette 1 may detect a smoking request by the user by detecting a suction (a puff) of the suction port 42 by the user with the suction sensor. In this case, the suction sensor is connected to the electronic control portion 22 via electric wiring, and when the suction sensor detects a suction (a puff) of the suction port 42 by the user, the electronic control portion 22 may control the battery 21 and cause the battery 21 to feed power to a porous heating element of the cartridge 3 to be described later. In addition, in the present invention, a pressure sensor which detects negative pressure created by the user's suction or a thermal type flowmeter (such as a MEMS flow sensor) can be used as the suction sensor. Furthermore, while the atomization cavity 45a is provided in the mouthpiece portion 4, the housing cavity 23 on the side of the main body portion 2 (the battery assembly) may be made deeper to enable the atomization cavity 45a to be provided in the main body portion 2. In this case, preferably, the air intake port 43 is also provided in the main body portion 2 (refer to Figs. 11A, 12A, and the like).

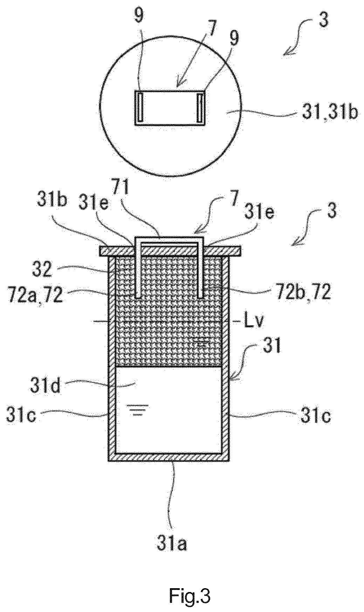

Fig. 3 is a diagram showing a schematic configuration of the cartridge 3 according to the present embodiment. An upper half shows the upper surface of the cartridge 3 and a lower half shows a vertical section of the cartridge 3. In the present embodiment in which the cartridge 3 internally has a liquid tank 31 for housing the aerosol-forming liquid, while the liquid tank 31 is, for example, a cylindrical bottle case having a circular bottom portion 31a, a circular lid portion 31b, and a cylindrical side wall surface 31c, a shape of the liquid tank 31 is not particularly limited. A liquid storage space 31d for storing the aerosol-forming liquid is formed inside the liquid tank 31, and the aerosol-forming liquid is stored inside the liquid storage space 31d. The aerosol-forming liquid may be, for example, a mixture of glycerin (G), propylene glycol (PG), a nicotine solution, water, a fragrance, and the like. A mixture ratio of the materials contained in the aerosol-forming liquid can be changed when appropriate. In the present invention, the aerosol-forming liquid may not contain a nicotine solution.

In addition, a liquid supplying member 32 which supplies the aerosol-forming liquid to a porous heating element to be described later is arranged on an upper side of the liquid storage space 31d in the liquid tank 31. For example, the liquid supplying member 32 may be a cotton fiber. In the present embodiment, the liquid supplying member 32 may be fixed to, for example, a rear surface of the lid portion 31b in the liquid tank 31. It should be noted that, in the present invention, the liquid supplying member 32 may not be provided. Reference numeral 7 in Fig. 3 denotes a porous heating element which atomizes the aerosol-forming liquid stored in the liquid tank 31 by heating the aerosol-forming liquid. In addition, reference character Lv in Fig. 3 exemplifies an initial liquid level of the aerosol-forming liquid stored in the liquid tank 31 (the liquid storage space 31d). When manufacturing the electronic cigarette 1, a liquid level of the aerosol-forming liquid is adjusted to the initial liquid level Lv by storing a prescribed amount of the aerosol-forming liquid in the liquid tank 31 (the liquid storage space 31d). By setting the initial liquid level Lv above the liquid supplying member 32 or, in other words, by filling the aerosol-forming liquid to above a lower end of the liquid supplying member 32, the aerosol-forming liquid can be supplied to the porous heating element in a stable manner.

The porous heating element 7 is folded in an approximate C-shape in a side view. When not in use, at least a part of the porous heating element 7 is in direct contact or in indirect contact via the liquid supplying member 32 with the aerosol-forming liquid inside the liquid tank 31 (the liquid storage space 31d). The porous heating element 7 is a wick cum heater which is equipped with both a function as a wick that directly or indirectly suctions and retains the aerosol-forming liquid stored in the liquid tank 31 and a function as a heater that atomizes the retained aerosol-forming liquid by electric heating when the user smokes. The porous heating element 7 includes a tabular heater portion 71 arranged so as to oppose a surface of the lid portion 31b of the liquid tank 31 and a first sucking portion 72a and a second sucking portion 72b which are folded downward from the heater portion 71. Hereinafter, when collectively referring to the first sucking portion 72a and the second sucking portion 72b, the sucking portions 72a and 72b will be referred to as a "sucking portion 72".

An insertion hole 31e for inserting the sucking portion 72 into the liquid tank 31 is formed in the lid portion 31b of the liquid tank 31, and the sucking portion 72 is inserted to a side of the liquid storage space 31d through the insertion hole 31e. While folding both sides of the heater portion 71 results in a pair of the sucking portions 72 being installed consecutively to the heater portion 71 in the present embodiment, the number of the sucking portions 72 is not particularly limited. It should be noted that, for example, as shown in Fig. 3, a tip of the sucking portion 72 may extend to inside of the liquid supplying member 32 constituted by a cotton fiber or may penetrate the liquid supplying member 32 and extend toward the side of the liquid storage space 31d. In the present invention, the respective members may be arranged so that a part of the sucking portion 72 comes into contact with a surface of the liquid supplying member. A contact area between the sucking portion 72 and the liquid supplying member 32 and a contact surface of the sucking portion 72 with the liquid supplying member 32 (for example, an upper end surface or a side peripheral surface of the liquid supplying member 32) can be changed as appropriate.

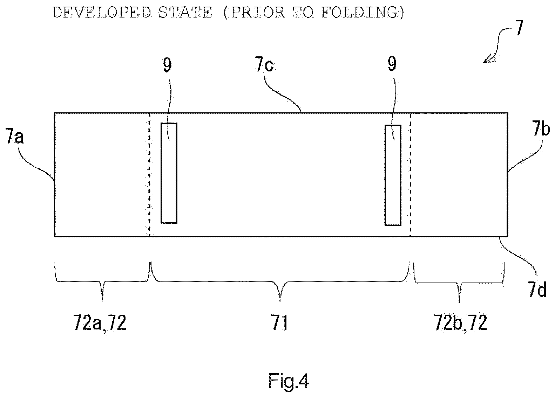

Fig. 4 is a diagram showing a planer structure of the porous heating element 7 according to the present embodiment. It should be noted that Fig. 4 shows a state where the porous heating element 7 has been developed or, in other words, a state prior to folding the sucking portion 72 with respect to the heater portion 71. Dash lines in the drawing indicate boundaries between the heater portion 71 and the sucking portions 72.

In the example shown in Fig. 4, the porous heating element 7 has a rectangular planar shape. The shape of the porous heating element 7 is not particularly limited and the porous heating element 7 may have a parallelogram shape, a rhomboid shape, or the like. Reference characters 7a, 7b, 7c, and 7d denote a left side, a right side, an upper side, and a lower side of the porous heating element 7. Hereinafter, a direction along the upper side 7c and the lower side 7d of the porous heating element 7 will be referred to as a lateral width direction of the porous heating element 7. In addition, a direction along the left side 7a and the right side 7b of the porous heating element 7 will be referred to as a vertical direction of the porous heating element 7.

The porous heating element 7 used in the present invention will be described in detail later.

<Second embodiment>

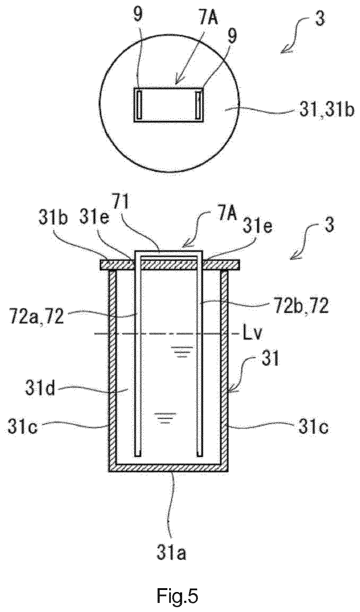

Fig. 5 is a diagram showing a cartridge 3A according to a second embodiment. In the cartridge 3A shown in Fig. 5, the liquid tank 31 (the liquid storage space 31d) is not provided with the liquid supplying member 32. In addition, a porous heating element 7A according to the second embodiment is configured such that the sucking portion 72 extends to a vicinity of a bottom portion of the liquid tank 31 and the sucking portion 72 directly sucks up the aerosol-forming liquid stored in the liquid storage space 31d.

<Third embodiment>

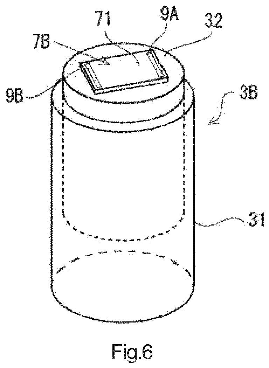

Fig. 6 is a diagram showing a cartridge 3B according to a third embodiment. A porous heating element 7B in the cartridge 3B shown in Fig. 6 is solely constituted by the heater portion 71 and does not have the sucking portion 72. In the cartridge 3B, for example, the liquid supplying member 32 formed in a columnar shape is provided in the liquid tank 31, and the porous heating element 7B is placed on the upper surface of the liquid supplying member 32. The heater portion 71 in the porous heating element 7B shares a same structure as the heater portion 71 of the porous heating element 7 according to the first embodiment. The porous heating element 7B according to the present embodiment can suction and retain the aerosol-forming liquid from a rear surface of the heater portion 71 which is in contact with the upper surface of the liquid supplying member 32. It should be noted that the shape of the liquid supplying member 32 is not limited to the example described above.

<Fourth embodiment>

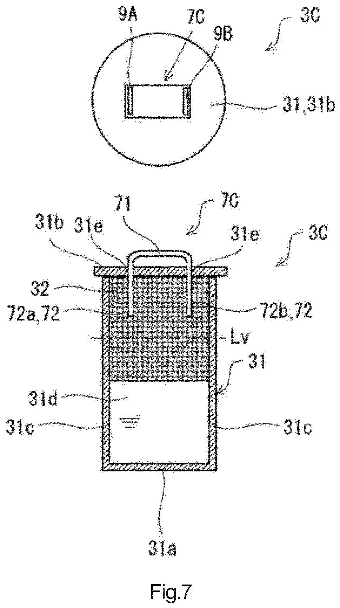

Fig. 7 is a diagram showing a cartridge 3C according to a fourth embodiment. Although a porous heating element 7C in the cartridge 3C differs from the porous heating element 7 according to the first embodiment which is folded in an approximate C-shape in a side view in that the porous heating element 7C has an approximate U-shape in a side view, other structures are the same.

<Fifth embodiment>

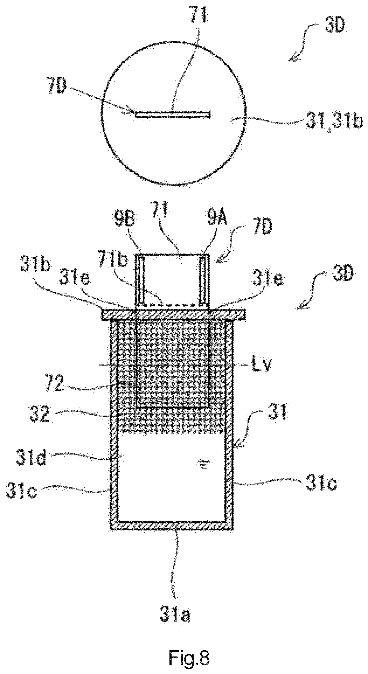

Fig. 8 is a diagram showing a cartridge 3D according to a fifth embodiment. In a porous heating element 7D in the cartridge 3D, a single sucking portion 72 is connected to the right side 71b of the heater portion 71. Other structures are the same as the porous heating element 7 according to the first embodiment.

The porous heating element 7D has a tabular shape as a whole, and the sucking portion 72 is inserted into the liquid storage space 31d through the insertion hole 31e formed in the lid portion 31b of the liquid tank 31. In other words, in the cartridge 3D, the porous heating element 7D with the tabular shape is installed in the liquid tank 71 in a mode where the heater portion 71 of the porous heating element 7D is exposed to the outside of the liquid tank 71 and the sucking portion 72 is inserted to the outside of the liquid tank 71.

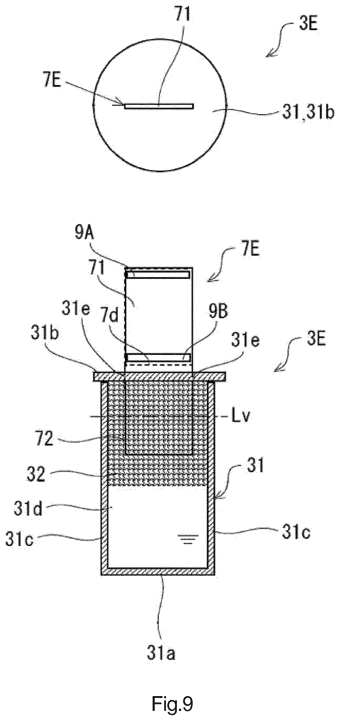

Fig. 9 is a diagram showing a cartridge 3E according to a first modification of the fifth embodiment. A porous heating element 7E provided in the cartridge 3E shares a same structure as the porous heating element 7D shown in Fig. 8 with the exception of a single sucking portion 72 being connected to the lower side 7d of the heater portion 71. The porous heating element 7E has a tabular shape as a whole. The porous heating element 7E has a tabular shape as a whole, and the sucking portion 72 is inserted into the liquid storage space 31d through the insertion hole 31e formed in the lid portion 31b of the liquid tank 31. In other words, in the cartridge 3E, the porous heating element 7E with the tabular shape is installed in the liquid tank 31 in a mode where the heater portion 71 of the porous heating element 7E is exposed to the outside of the liquid tank 31 and the sucking portion 72 is inserted to the outside of the liquid tank 31.

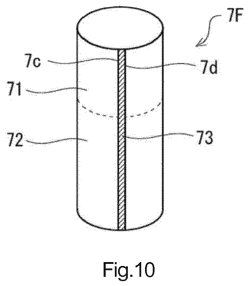

In addition, Fig. 10 is a diagram showing a porous heating element 7F according to a second modification of the fifth embodiment. In the porous heating element 7F, a single sucking portion 72 is connected to the right side 7b of the heater portion 71 and the porous heating element 7F is rounded so as to form a cylindrical shape. In the illustrated example, an insulating member 73 is provided between the upper side 7c and the lower side 7d of the heater portion 71, and the upper side 7c and the lower side 7d of the heater portion 71 are insulated by the insulating member 73. It should be noted that a positive electrode 9A, a negative electrode 9B, and the like in the heater portion 71 are not shown in Fig. 10. Alternatively, instead of interposing the insulating member 73 between the upper side 7c and the lower side 7d of the heater portion 71 in the porous heating element 7F, the porous heating element 7F may be rounded in a C-shape so that a gap is formed between the upper side 7c and the lower side 7d.

<Sixth embodiment>

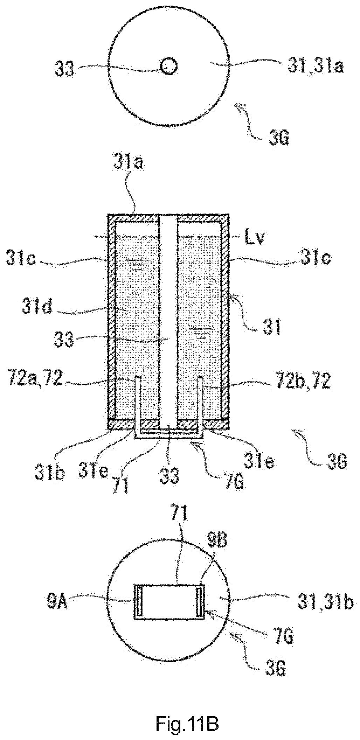

Fig. 11A is a diagram showing an electronic cigarette 1G according to a sixth embodiment. Fig. 11B is a diagram showing a cartridge 3G according to the sixth embodiment. The cartridge 3G has the porous heating element 7 described with reference to Fig. 4. The liquid tank 31 in the cartridge 3G has an annular shape and a hollow through-passage 33 is provided in a central part of the liquid tank 31. As illustrated, the hollow through-passage 33 of the liquid tank 31 in the cartridge 3G penetrates the liquid tank 31 in the vertical direction. The porous heating element 7 comes into contact with the aerosol-forming liquid by inserting the sucking portion 72 into the liquid storage space 31d through the insertion hole 31e provided in the lid portion 31b of the liquid tank 31 in a similar manner to the first embodiment.

The cartridge 3G is housed in the housing cavity 23 so that the lid portion 31b of the liquid tank 31 faces a far side (an inner side) of the housing cavity 23. Specifically, the cartridge 3G according to the sixth embodiment is housed in the housing cavity 23 so as to be vertically inverted with respect to the cartridge 3 according to the first embodiment. In other words, the cartridge 3G is arranged so that a side of the bottom portion 31a of the liquid tank 31 faces the mouthpiece portion 4. In the electronic cigarette 1G, the air intake port 43 is provided on the main body-side housing 20 in the main body portion 2, and air taken into the main body-side housing 20 from the outside through the air intake port 43 travels along the hollow through-passage 33 and the internal passage 45 of the mouthpiece portion 4 together with an aerosol formed in the porous heating element 7 in the cartridge 3G and reaches the suction port 42, thereby enabling the user to suction the aerosol from the suction port 42.

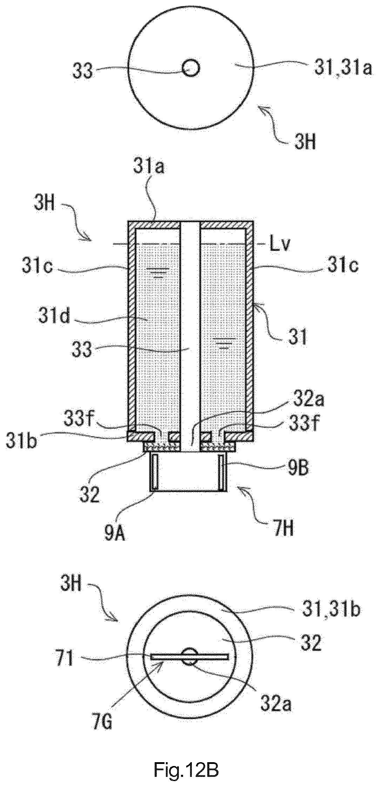

Fig. 12A is a diagram showing an electronic cigarette 1H according to a modification of the sixth embodiment. Fig. 12B is a diagram showing a cartridge 3H according to the modification of the sixth embodiment. In the cartridge 3H, the liquid tank 31 has an annular shape provided with the hollow through-passage 33 on a central side in a similar manner to the cartridge 3G. The liquid supplying member 32 made of, for example, a cotton fiber is arranged on an outer surface side of the lid portion 31b of the liquid tank 31 in the cartridge 3H. The liquid supplying member 32 has a disk shape and has a vent hole 32a at a position corresponding to the hollow through-passage 33 of the liquid tank 31. In addition, a liquid supply hole 33f for supplying the aerosol-forming liquid stored in the liquid tank 31 (the liquid storage space 31d) to the liquid supplying member 32 is provided in the lid portion 31b of the liquid tank 31.

The cartridge 3H according to the present embodiment has a porous heating element 7H which is solely constituted by the heater portion 71 and which shares a same structure as the porous heating element 7B according to the third embodiment. In the example shown in Fig. 12B, the porous heating element 7H is fixed to the liquid supplying member 32 in a state where an end surface of the porous heating element 7H abuts against an outer surface of the liquid supplying member 32. With the electronic cigarette 1H configured as described above, the aerosol-forming liquid stored in the liquid tank 31 (the liquid storage space 31d) of the cartridge 3H is supplied to the porous heating element 7H (the heater portion 71) through the liquid supplying member 32 and retained by the heater portion 71. Subsequently, when a current is passed between electrodes of the heater portion 71, the aerosol-forming liquid retained by the heater portion 71 is atomized to form an aerosol.

In addition, as shown in Fig. 12A, in the electronic cigarette 1H, the air intake port 43 is provided on the main body-side housing 20 in the main body portion 2, and air taken into the main body-side housing 20 from the outside through the air intake port 43 travels through the vent hole 32a of the liquid supplying member 32, the hollow through-passage 33 of the liquid tank 31, and the internal passage 45 of the mouthpiece portion 4 together with an aerosol formed in the porous heating element 7H (the heater portion 71) and reaches the suction port 42, thereby enabling the user to suction the aerosol from the suction port 42.

<Porous heating element>

The porous heating element 7 described above and used in the present invention is a porous heating element which is capable of at least temporarily retaining an aerosol-forming liquid and which has a function of suctioning a liquid as well as a function of heating the liquid.

With the porous heating element 7 used in a cartridge of the flavor inhaler according to the present invention, a relative standard deviation of resistance values measured under the following test conditions is 5.0% or less.

Test conditions : A positive electrode and a negative electrode are arranged on a porous heating element so as to create an 8 mm-long electrical path, and a resistance value is measured a total of 30 times. The measurements of the resistance value are performed by changing a position of the positive electrode or the negative electrode for each measurement so as to maintain the 8 mm length of the electrical path.

Configuring the porous heating element 7 so that the relative standard deviation of the resistance values measured under the test conditions described above is 5.0% or less enables localized heat generation when voltage is applied to be suppressed and, as a result, uniform heat generation can be realized. This is also obvious from the fact that, since an amount of heat generation Q is given by $Q = I^2 \times R \times t$ (where I: current, R: resistance, and t: time) based on Ohm's law and Joule's law, a small variation in R indicates a small variation in amounts of heat generation.

A heating element used in a general electronic cigarette is formed by winding a coil around a glass fiber called a wick, in which case the wick performs liquid absorption while the coil performs atomization of the liquid retained in the wick. In order to secure a contact area between the wick and the coil, a coil with an electrical path length of approximately 20 mm or longer is generally used.

Deliberations by the present inventors revealed that the electrical path length of a porous heating element is approximately 20 mm or longer even when the porous heating element is used to atomize an aerosol-forming liquid.

This indicates that the 8 mm-distance of the electrical path in the test conditions set forth in the present invention is an extremely short distance.

When measuring a resistance value of a given substance, usually, electrodes are arranged at an end part and another end part of the substance or, in other words, arranged so as to approximately maximize a length of an electrical path between a positive electrode and a negative electrode. For example, in the case of a heating element which is solid and which has a uniform internal structure including a general heating element such as a coil, a variation in resistance values acquired by the method described above (over an entire length of the heating element) and a variation in resistance values acquired with respect to an electrical path distance of 8 mm are approximately the same. On the other hand, when a heating resistor is a porous body, a variation in resistance values acquired by the method described above (over an entire length of the heating element) and a variation in resistance values acquired with respect to an electrical path distance of 8 mm may differ from each other. This is because sizes, a variation, and the like of pores of a porous heating element affect the resistance values.

In such a porous heating element, by acquiring a variation in resistance values with respect to an electrical path length of 8 mm (shorter than a length of an approximately maximum electrical path of the porous heating element) a plurality of times while changing locations of the positive electrode and the negative electrode, whether or not there is a location where resistance of the porous heating element is locally high can be verified.

Deliberations by the present inventors revealed that, when the length of the electrical path is set to 8 mm, a variation in resistances is small even in an extremely small area of a porous heating element if a relative standard deviation or, in other words, a variation among respective resistances is 5.0% or less and, therefore, when the porous heating element is used to atomize an aerosol, uniform heating can be perform over the entire porous heating element.

As for the measurement of resistance values, the number of measurements is set to 30 times. By increasing the number of measurements while changing locations to be measured, reliability of test results (in particular, relative standard deviation) can be increased. For measurement points of a sample during the 30 measurements, a position of the positive electrode or the negative electrode is moved so that measurements are performed over the entire sample.

In addition, when measuring resistance of a porous heating element, conditions of a sample used in the test are to be set the same. Specifically, the test described above is performed to calculate relative standard deviation while ensuring the porous heating element is not bent and a slit or the like is not created along the electrical path.

Furthermore, when measuring resistance of a porous heating element, a tabular porous heating element is used.

It should be noted that, in the present invention, when a porous heating element is actually used in a cartridge of a flavor inhaler of an electronic cigarette, varying resistance values by bending the porous heating element or creating a slit is permissible.

In addition, dimensions of a porous heating element used in a cartridge of a flavor inhaler are not directly related to a size of a sample when used in the test described above. In other words, dimensions of a sample used in the test described above may differ from dimensions of the porous heating element used in a cartridge of a flavor inhaler.

Furthermore, when localized heat generation does not occur in a porous heating element, it is expected that a bias is not created in atomization of a liquid containing a flavor source and the like when using a flavor inhaler and a decline in flavor due to excessive localized heating of the liquid can be suppressed.

When the relative standard deviation of the resistance values described above exceeds 5.0%, localized heat generation may occur in a porous heating element.

A porous heating element of which resistance values have a relative standard deviation of 5.0% or less can be obtained using, for example, a manufacturing method to be described later.

While an apparent volume resistivity of a porous heating element is not particularly limited as long as a property of performing resistance heating when being applied voltage of approximately 1 to 10 V and preferably approximately 3 to 4 V is exhibited with respect to a prescribed shape and prescribed dimensions, for example, the apparent volume resistivity ranges from $1 \times 10^{-9}$ to $1 \times 10^{8}$ Ω·m and preferably ranges from $1 \times 10^{-7}$ to $1 \times 1 \times 10^{-3}$ Ω·m. Apparent volume resistivity refers to apparent resistivity derived from dimensions including pores and can be calculated as resistance value $\times$ (width $\times$ length)/ length.

In the present invention, a material constituting a porous heating element is not particularly limited as long as the material is capable of generating heat when power is applied thereto and exhibits a property obtained by measurement under the test conditions described earlier.

Examples of the material constituting a porous heating element include a porous metal body made of nickel, nichrome, or SUS (stainless steel), but ceramics can also be used.

The porous heating element preferably at least contains nickel (hereinafter, also described as Ni) and chromium (hereinafter, also described as Cr).

When the porous heating element is a porous metal body containing nickel and chromium, a weight ratio of nickel and chromium in the porous metal body is, for example, 20:80 to 90:10 and preferably 60:40 to 85:15.

On the other hand, when the porous heating element is a ceramic, a porous heating element made of silicon carbide can be exemplified.

In addition, the porous heating element 7 used in the present invention has a three-dimensional network structure. The three-dimensional network structure contains pores and has a structure in which at least a part of the pores are communicated with each other or, in other words, an open-cell structure.

A size of the pores can be expressed as a nominal pore diameter, and the nominal pore diameter of a porous heating element used in the present invention is preferably 1000 μm or less and more preferably 500 μm or less.

While a lower limit value of the nominal pore diameter is not particularly limited as long as the open-cell structure is maintained (as long as the pores are not crushed and become blocked), for example, the lower limit value can be set to 100 μm or more.

With the porous heating element 7 included in the flavor inhaler according to the present invention, uniformity of a pore distribution inside the porous heating element 7 is conceivably high. As will be described later, whether or not pores uniformly exist in a porous heating element can be checked by performing an operation involving arranging electrodes at specific width intervals in the porous heating element and measuring a resistance value 30 times to acquire a plurality of resistance values and by calculating a relative standard deviation of the resistance values.

A shape and dimensions of a porous heating element are not particularly limited as long as the porous heating element can be housed in a cartridge of the flavor inhaler.

A volume (including pores) of a porous heating element is, for example, 1 to 300 mm$^3$ and preferably 3 to 50 mm$^3$. The volume of the porous heating element containing internal pores is adjusted in accordance with an amount in which a liquid to be atomized is retained, resistivity, and a target resistance value.

A thickness of a porous heating element used in the flavor inhaler according to the present invention when mounted is, for example, 0.2 to 2.0 mm and preferably 0.2 to 1.0 mm. The thickness of the porous heating element is adjusted in accordance with an amount in which a liquid to be atomized is retained, resistivity, and a target resistance value.

In addition, for example, a porous heating element can be molded in a sheet form (a tabular) form in a shape such as a rectangle or a square.

When the porous heating element is a rectangle, a length in a longitudinal direction thereof is, for example, 5 to 50 mm and preferably 10 to 40 mm. On the other hand, a length in a transverse direction thereof is, for example, 0.1 to 30 mm and preferably 0.5 to 10 mm.

Porosity of a porous heating element is, for example, 50% or higher, preferably 60% or higher, and more preferably 70% or higher.

Furthermore, with the porous heating element 7 applied to a flavor inhaler cartridge, an electrical path length is preferably 20 mm or longer and 80 mm or shorter and more preferably 30 mm or longer and 60 mm or shorter. As a method of extending the electrical path length without changing the length in the longitudinal direction described above, a method of providing a slit between the electrodes and causing the electrical path to meander can be favorably used.

It should be noted that an electrical path length according to the present invention refers to a distance between electrodes between which a current is assumed to flow in the porous heating element 7.

Meanwhile, while an upper limit of porosity is not particularly limited, for example, the upper limit of porosity is 99% or lower.

The porosity can be measured in conformity with JIS Z 2501:2000 (ISO/DIS 2738: 1996).

In addition, in the porous heating element 7, a total area of a portion that functions as a heater preferably ranges from 1 to 250 mm$^2$ and more preferably ranges from 3 to 150 mm$^2$. Furthermore, when the porous heating element 7 is a rectangle, an aspect ratio (long side: short side) of the portion that functions as a heater preferably ranges from 1:1 to 3:1 and more preferably ranges from 1:1 to 2:1.

Can be performed by.

The porous heating element included in the flavor inhaler according to the present invention can be fabricated using, for example, a first manufacturing method shown below and described in Japanese Patent Application Laid-open No. 2012-149282 and Japanese Patent Application Laid-open No. H08-232003, a second manufacturing method described in Japanese Patent Application Laid-open No. 2003-119085, or an improvement of these methods.

With these manufacturing methods, using a porous resin having a three-dimensional network structure molded in advance as a skeleton when forming the porous heating element results in an internal structure of the obtained porous heating element being reflective of a pore distribution inside the porous resin. Therefore, a porous heating element which internally has a uniform pore distribution can be obtained by using a porous resin which internally has a uniform pore distribution.

Accordingly, in the porous heating element, uniformity of the pore distribution in an extremely small area such as 8 mm-intervals increases and a localized variation in resistance values is reduced.

Uniform pore distribution in the porous heating element is a property not obtainable with a manufacturing method of a SUS (stainless steel) 316 slurry foam used in a comparative example to be described later. In the manufacturing method of the SUS 316 slurry foam used in the comparative example, steps of forming a porous structure with a resin and obtaining a porous metal body from metal powder are simultaneously performed without using a porous resin such as that described above. While a slurry containing a resin and metal powder is used as a raw material in the manufacturing method of the SUS 316 slurry foam, it is difficult to form a slurry in which these components are uniformly mixed. Therefore, when a skeleton of a porous metal body is formed in a step near the end of a manufacturing process as in the manufacturing method of the SUS 316 slurry foam, a pore distribution inside the obtained porous metal body is conceivably not uniform.

It should be noted that a foam material is preferably not used (not added) during steps in both the first manufacturing method and the second manufacturing method to be described below. This is because using a foam material may prevent a uniform pore distribution from being obtained inside the porous heating element.

<First manufacturing method>

An example of the first manufacturing method is a manufacturing method at least including: a step of applying a conductivity-imparting process to a porous resin; a first plating step of applying metal plating to the porous resin having been subjected to the conductivity-imparting process; and a heat treatment step of removing the porous resin.

Examples of this manufacturing method include the methods described in Japanese Patent Application Laid-open No. 2012-149282 and Japanese Patent Application Laid-open No. H08-232003.

It should be noted that, when a porous heating element is a porous metal body containing a plurality of types of metal such as nickel and chromium, a second plating step of applying plating with a metal that differs from the metal used in the first plating step can be included after the heat treatment step described above.

An example of a porous heating element manufactured by the manufacturing method described above is Celmet (trade name) manufactured by Sumitomo Electric Industries, Ltd. The trade name Celmet includes a product lineup of a plurality of porous metal bodies including a porous metal body containing nickel and a porous metal body containing a nichrome alloy.

The step of applying a conductivity-imparting process to a porous resin is a step of providing a conductive coating layer on a surface of a porous body made of resin. While a resin foam, a non-woven fabric, felt, a woven fabric, or the like is used as the porous resin, these materials may be used in combination if necessary. In addition, while the material is not particularly limited, a material that can be removed by an incineration process after performing metal plating is preferable.

In the present invention, a resin foam is preferably used as the porous resin. Well-known or commercially available resin foams can be used as long as porosity is provided, and examples include foamed urethane and Styrofoam. Among such resin forms, foamed urethane is preferable from the perspective of its particularly high porosity. A thickness, porosity, and an average pore diameter of the foam-like resin are not restrictive and can be appropriately set according to application. The foamed urethane can be formed by fabricating a urethane foam having open-cells as follows: after performing vacuuming inside an explosion-proof chamber, subjecting the inside of the chamber to vacuum displacement using a gas with a mixing ratio of hydrogen: oxygen = 2:1; and igniting the gas to break most of bubbles inside the urethane foam with the blast (a film-removing process).

In addition to a resin foam such as that described above, the porous resin can also be fabricated using a 3D printer.

Examples of the material constituting the conductive coating layer include metals such as nickel, titanium, and stainless steel as well as carbon black. As a specific example of the conductivity-imparting process, for example, when nickel is used as the material constituting the conductive coating layer, an electroless plating process, a sputtering process, and the like can be used. In addition, when materials such as metals including titanium and stainless steel, carbon black, black lead, and the like are used, a process can be performed in which a mixture obtained by adding a binder to a fine powder of these materials is applied to a surface of a resin porous body.

The conductive coating layer need only be continuously formed on a surface of the resin porous body, and while a coating weight thereof is not restrictive, a normal coating weight is around 0.1 g/m² or more and 20 g/m² or less.

The first plating step of applying metal plating to the porous resin is not particularly limited as long as the step involves applying metal plating by a known plating method and, for example, electroplating can be used.

Examples of the metal used in the first plating step include nickel and chromium, and nickel is preferably used.

When using nickel, a nickel electroplating layer need only be formed on the conductive coating layer so as not to expose the conductive coating layer, and while a coating weight thereof is not restrictive, a normal coating weight is around 100 g/m² or more and 600 g/m² or less.

An example of the heat treatment step of removing the porous resin is a step of performing a heat treatment at 600°C or higher and 800°C or lower under an oxidizing atmosphere such as atmospheric air inside a stainless steel muffle.

When the porous heating element used in the present invention contains a plurality of types of metal, a second plating step of applying plating with a metal that differs from the metal used in the first plating step may be included after the heat treatment step.

As the metal used in the second plating step, for example, when nickel is plated in the first plating step, chromium can be used in the second plating step as the metal to be plated.

In this case, the second plating step of applying Cr plating on a Ni layer is not particularly limited as long as the step involves applying chromium plating by a known plating method and, for example, electroplating can be used.

A coating weight of a chromium electroplating layer is not restrictive and a normal coating weight is around 10 g/m² or more and 600 g/m² or less.

When the porous heating element used in the present invention includes two types of metal such as nickel and chromium, a second heat treatment step described below may be included.

An example of the second heat treatment step of alloying a Ni layer and a Cr layer is a step of performing a heat treatment at 800°C or higher and 1100°C or lower under a reducing gas atmosphere such as CO or $H_2$ inside a stainless steel muffle.

<Second manufacturing method>

When the porous heating element 7 uses a ceramic as a raw material, an example of the ceramic is silicon carbide (SiC) and, for example, a step described below may be included in the second manufacturing method of a porous heating element.

The second manufacturing method of a porous heating element includes: a step of retaining a slurry obtained by mixing a carbon source with silicon powder on a skeleton of a porous structure and subsequently drying the slurry; a first heating step of heating the dried porous structure under vacuum or under an inert atmosphere to obtain a porous composite; a second heating step of heating, under vacuum or under an inert atmosphere, the porous composite obtained in the first heating step to obtain a porous sintered body; and a third heating step of heating the obtained porous sintered body under vacuum or under an inert atmosphere such as argon to obtain the porous heating element.

As a manufacturing method of a porous heating element constituted by silicon carbide, for example, Japanese Patent Application Laid-open No. 2003-119085 can be used as a reference.

<Step of retaining slurry on skeleton of porous resin>

The step of retaining a slurry on a skeleton of a porous structure is a step of either applying a slurry obtained by mixing a carbon source constituted by a resin such as phenolic resin with silicon powder to a tangible skeleton of a stretchable porous resin or impregnating a porous structure with the slurry to retain the slurry on the skeleton of the porous structure. Subsequently, the porous structure is squeezed and dried to such a degree that open-cell portions are not blocked by the slurry liquid. Examples of the porous resin include a sponge made of resin, rubber, or the like or plastics with a sponge form.

<First heating step>

The first heating step is a step of heating the dried porous structure at, for example, around 900°C to 1350°C under vacuum or inert atmosphere such as argon. Accordingly, a porous composite is obtained. The skeleton portion that constitutes the porous resin is in a state where a carbon portion constituted by a resin such as phenolic resin and silicon powder are mixed with each other.

As the silicon powder, silicon powder of approximately 30 μm or smaller can be used.

<Second heating step>

The second heating step is a step of heating the porous composite obtained in the first heating step at a temperature of, for example, 1350°C or higher under vacuum or under an inert atmosphere such as argon, and reacting carbon with silicon and forming porous silicon carbide with favorable wettability to molten silicon on a tangible skeleton portion of porous resin to obtain a porous sintered body.

Since this reaction is a volume-reducing reaction, open pores attributable to the volume-reducing reaction are created. Accordingly, a porous sintered body of which a matrix portion is formed by silicon carbide having pores and residual carbon is obtained.

<Third heating step>

The third heating step is a step of heating the porous sintered body obtained in the second heating step to, for example, around 1300°C to 1800°C under vacuum or under an inert atmosphere such as argon. Due to this step, by impregnating the porous silicon carbide and the carbon portion on the skeleton with molten silicon, a porous heating element constituted by silicon carbide is obtained.

An example of a mixing ratio of the silicon powder and the carbon source such as resin used in the method described above is an atomic ratio of silicon and carbon expressed as Si/C = 0.05 to 4.

With a porous heating element obtained by the manufacturing method exemplified above, uniformity of an internal pore distribution of the porous resin is conceivably high. The use of a porous heating element of which uniformity of internal pores is conceivably high in a cartridge of a flavor inhaler is something that has never been done before.

It is difficult to accurately express physical properties of the porous heating element used in the present invention other than stating that resistance values of the porous heating element has a relative standard deviation in the specific range described earlier. Therefore, instead of expressing the porous heating element in terms of physical properties, the present specification indicates that fabricating a porous heating element by the manufacturing method described above contributes to imparting the physical properties of the porous heating element used in the present invention.

While preferred embodiments of the present invention have been described above, it should be obvious to those skilled in the art that various modifications, improvements, combinations and the like can be made to the flavor inhaler according to the present invention and to a cartridge and a porous heating element applied to the flavor inhaler according to the present invention.

Examples

While the present invention will be described with further specificity using examples, it is to be understood that the present invention is not limited to the descriptions of the following examples insofar as the gist of the present invention is not exceeded.

<Examples 1 to 3>

A 1 mm-thick sample of trade name: Celmet (manufactured by Sumitomo Electric Industries, Ltd.; a porous metal containing a nickel-chromium alloy in which nickel: chromium = 80:20 and porosity: approximately 95%) with a nominal pore diameter of 450 μm was cut by laser to a 1.5 mm width. It should be noted that Celmet was fabricated using the manufacturing method of a porous heating element described above. Three samples of Celmet with different apparent volume resistivities were prepared by adjusting the manufacturing method (adjusting the Cr plating step).

<Comparative examples 1 and 2>

Two types (with different nominal pore diameters and different porosities (Comparative example 1: approximately 90%, Comparative example 2: approximately 82%)) of a 2 mm-thick SUS 316 slurry foam (manufactured by Mitsubishi Materials Corporation) was used.

The SUS slurry foams were fabricated by a method roughly involving heating a slurry-like substance obtained by mixing a metal material precursor with a foam material to obtain a metal material (stainless steel) having a foam structure instead of the manufacturing method of a porous heating element described above.

Each sample of the Examples 1 to 3 and the Comparative examples 1 and 2 was cut and compressed by a vise to obtain an approximately rectangular sample with a length in the longitudinal direction of approximately 40 mm, a length in the transverse direction of approximately 1.0 to 1.5 mm, and a thickness of approximately 0.2 to 0.5 mm.

Dimensions and physical properties of the respective materials are listed in Table 1.

[Table 1]

Table 1

| Material | Celmet_1 | Celmet_2 | Celmet_3 | SUS_1 | SUS_2 |
|---|---|---|---|---|---|
| Apparent volume resistivity ($\Omega \cdot$m) | $1.3 \times 10^{-5}$ | $1.9 \times 10^{-5}$ | $3.8 \times 10^{-5}$ | - | - |
| Pore diameter ($\mu$m) | 450 | 450 | 450 | 600 | 150 |
| Length (mm) | 40 | 40 | 40 | 40 | 40 |
| Width (mm) | 1.5 | 1.0 | 1.4 | 1.5 | 5.2 |
| Thickness (mm) | 0.2 | 0.5 | 0.5 | 0.2 | 0.2 |

```
* Apparent volume resistivity = apparent resistivity derived from dimensions including pores = resistance value × (width × length)/

length
```

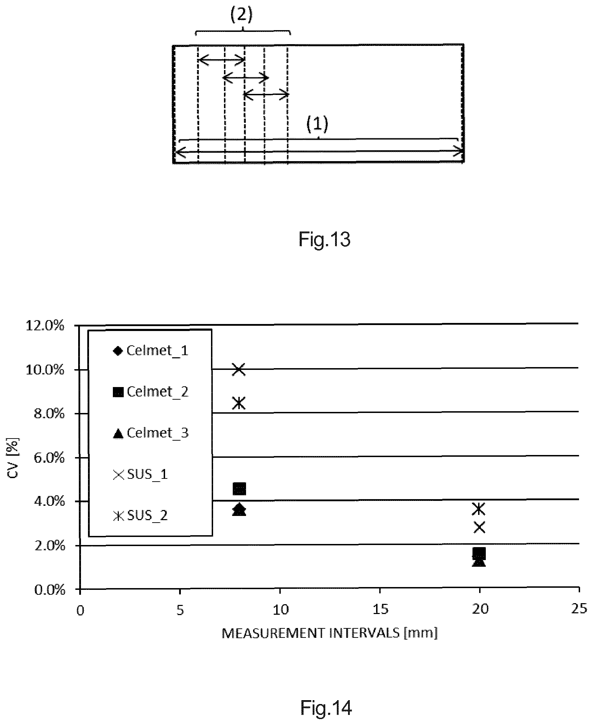

Measuring electrodes were clamped to each obtained sample so that, as indicated by dash lines in Fig. 13, the measuring electrodes came into contact with the sample at prescribed intervals (a length of both arrows) over an entire length in the transverse direction. Specifically, resistance values were measured by changing electrode positions so that measurement intervals were 8 mm and 20 mm. With respect to the resistance values at the measurement intervals of 8 mm and 20 mm, the entire sample was scanned by shifting a position of the electrodes in the longitudinal direction or the transverse direction so as to obtain measurement results at 30 points for each measurement interval.

It should be noted that randomness of a spatial structure of a porous heating element may be manifested as a variation in resistance values.

Although a measurement of resistance from one end to the other end of a porous heating element results in a resistance value of the porous heating element as a whole ((1) in Fig. 13), reducing a distance between the electrodes enables a degree of a localized variation of resistance values to be determined ((2) in Fig. 13).

Based on the respective pieces of obtained measurement data, an average value and a standard deviation of resistance values for each measurement interval of each material were obtained. Furthermore, based on the obtained average value and the standard deviation, a relative standard deviation (CV = standard deviation/ average value) of the resistance values was calculated. Results of the relative standard deviations are shown in Table. 2.

[Table 2]

Table 2

| Measurement intervals (mm) | Resistance value CV, % | | | | |
|---|---|---|---|---|---|
| | Celmet_1 | Celmet_2 | Celmet_3 | SUS_1 | SUS_2 |
| 8 | 3.6% | 4.5% | 3.6% | 10.0% | 8.4% |
| 20 | 1.4% | 1.6% | 1.3% | 2.8% | 3.6% |

A graph was created in which an ordinate represents obtained relative standard deviations and an abscissa represents measurement intervals. A result thereof is shown in Fig. 14.

The relative standard deviation obtained in the test described above is a value which expresses, in %, a variation in the resistance values at the measurement intervals. In other words, the larger the relative standard deviation, the larger the variation in the resistance values at the measurement intervals.

In the present test, there were differences among the samples with respect to the relative standard deviations at the measurement intervals of 8 mm and 20 mm. Specifically, while the relative standard deviation increased abruptly in the samples of the Comparative examples 1 and 2 when the measurement intervals were short, an increase in the relative standard deviation was suppressed in the samples of the Examples 1 to 3. This indicates that, while the variation in resistance values at short measurement intervals is large in the samples of the Comparative examples 1 and 2, the variation in resistance values at short measurement intervals is small in the samples of the Examples 1 to 3.

<Conceivable factors that may affect CV value other than uniformity of internal pore distribution>

After deliberations, the present inventors surmise that the differences in results between the examples and the comparative examples described above are attributable to the uniformity of internal pore distributions. On the other hand, the relative standard deviation of the resistance values described above may be affected by various factors. In consideration thereof, a contribution degree of factors other than the uniformity of a three-dimensional network structure to the relative standard deviation of resistance values was studied in order to indirectly demonstrate that the differences in results between the examples and the comparative examples described above are largely attributable to the uniformity of internal pore distributions.

(Pore diameter)

Table 2 clearly shows that the resistance CV value of the SUS sample with a pore diameter of 150 $\mu$m is smaller than that of the SUS sample with a pore diameter of 600 $\mu$m but larger than the Celmet sample with a pore diameter of 450 $\mu$m.
In other words, while the results between the SUS samples with different pore diameters suggest a trend in which the smaller the pore diameter size, the lower the relative standard deviation of resistance values, since the relative standard deviation of the Celmet sample with a pore diameter of 450 $\mu$m is lower than that of the SUS sample with a pore diameter of 150 $\mu$m, it is revealed that the pore diameter size is not a dominant factor in the differences in results between the examples and the comparative examples described above.

(Thickness)

Celmet_1 and Celmet_3 have different thicknesses of 0.2 mm and 0.5 mm, respectively. On the other hand, there were no significant variations in the CV values between the samples (Table 1 and Table 2).
In other words, it is revealed that the thickness of a porous heating element is not a dominant factor at least with respect to the differences in results between the examples and the comparative examples described above.

(Dimensional errors of samples)

As described earlier, the samples according to the present examples and the present comparative examples have an approximately rectangular sample with a length in the longitudinal direction of approximately 40 mm, a length in the transverse direction (a width) of approximately 1.0 to 1.5 mm, and a thickness of approximately 0.2 to 0.5 mm.
In order to examine a dimensional variation (a dimensional CV) upon creation of the samples, the length in the transverse direction (the width) and the thickness were measured 15 times while shifting positions in the longitudinal direction to measure variations in dimensions (dimensional CVs) of the samples. Results thereof are shown in Table 3.

[Table 3]

Table 3

|  | Dimension CV, % | |
|---|---|---|
|  | Celmet_2 | SUS_2 |
| Width | 2.10% | 1.00% |
| Thickness | 10.70% | 8.90% |

Meanwhile, Table 2 clearly shows that the Celmet with a larger dimensional variation has a lower relative standard deviation than SUS with a small relative standard deviation. In other words, it is revealed that the dimensional variation described above is not a dominant factor at least with respect to the differences in results between the examples and the comparative examples described above.

(Resistance measurement error)

When measuring electric resistance values 30 times at a fixed measurement position, CV values of Celmet and SUS were more or less the same (refer to Table 4 below).

[Table 4]

Table 4

| Resistance value CV, % | |
|---|---|
| Celmet_1 | SUS_2 |
| 1.00% | 1.10% |

In other words, it is revealed that the effect of variations in the resistance values attributable to a measurement device and a measurement method on relative standard deviation is extremely small.
Based on the results presented above and on deliberations, it is revealed that the factors described above do not have a dominant effect on the differences in relative standard deviations of resistance values among the examples and the comparative examples shown in Table 2. Therefore, albeit indirectly, it is revealed that the difference in uniformity of pore distributions is a main factor of the differences in effects among the examples and comparative examples.
When, after dropping 10 ml of a liquid constituting a 1:1 mixture of glycerin: propylene glycol on the sample (distance between electrodes: 40 mm) according to the Example 1, voltage of 3.5 V was applied between the electrodes, white smoke was visually observed in the Example 1.
In addition, when voltage was applied to the samples (distance between electrodes: 40 mm) of the Example 1 and the Comparative example 1 without retaining the liquid and a heating operation was performed, a visual observation of behaviors of the samples revealed that, while both samples eventually became entirely red-hot, there was a difference in the behaviors prior to reaching the red-hot state. Specifically, while the sample of the Example 1 became red-hot in a uniform manner, red-hot areas were locally observed on the sample of the Comparative example 1 before the entire sample became red-hot. In other words, it was confirmed that heat generation occurs more uniformly in the sample of the Example 1 as compared to the sample of the Comparative example 1.

[Reference Signs List]

1      Electronic cigarette
2      Main body portion
21     Battery
22     Electronic control portion
24     Housing cavity
3      Cartridge
31     Liquid tank
32     Liquid supplying member
4      Mouthpiece portion
42     Suction port
5      Hinge
7      Porous heating element
71     Heater portion
72     Sucking portion
9      Electrode
9A     Positive electrode
9B     Negative electrode

**Claims**

1.  A flavor inhaler cartridge, comprising:

    a liquid storage portion which stores an aerosol-forming liquid; and
    a porous heating element which is provided with a positive electrode and a negative electrode and which atomizes the aerosol-forming liquid supplied from the liquid storage portion by generating heat when a current is passed between the positive electrode and the negative electrode, wherein
    a relative standard deviation of resistance values of the porous heating element as measured under the following test conditions is 5.0% or less
    test conditions: the positive electrode and the negative electrode are arranged on the porous heating element so as to create an 8 mm-long electrical path, and a resistance value is measured a total of 30 times, the measurements of the resistance value being performed by changing a position of the positive electrode or the negative electrode for each measurement so as to maintain the 8 mm length of the electrical path.

2.  The flavor inhaler cartridge according to claim 1, wherein a nominal pore diameter of the porous heating element is 1000 $\mu$m or less.

3.  The flavor inhaler cartridge according to claim 1, wherein a porosity of the porous heating element is 50% or more.

4.  The flavor inhaler cartridge according to any one of claims 1 to 3, wherein the porous heating element is fabricated by a manufacturing method including: a step of applying a conductivity-imparting process to a porous resin; a first plating step of applying metal plating to the porous resin having been subjected to the conductivity-imparting process; and a heat treatment step of removing the porous resin.

5.  The flavor inhaler cartridge according to claim 4, wherein the metal used in the first plating step is nickel or chromium.

6.  The flavor inhaler cartridge according to claim 4 or 5, including, after the heat treatment step, a second plating step of applying plating with a metal that differs from the metal used in the first plating step.

7.  The flavor inhaler cartridge according to any one of claims 1 to 3, wherein the porous heating element includes:

    a step of retaining a slurry obtained by mixing a carbon source with silicon powder on a skeleton of a porous structure and subsequently drying the slurry;
    a first heating step of heating the dried porous structure under vacuum or under an inert atmosphere to obtain a porous composite;
    a second heating step of heating, under vacuum or under an inert atmosphere, the porous composite obtained in the first heating step to obtain a porous sintered body; and
    a third heating step of heating the obtained porous sintered body under vacuum or under an inert atmosphere to obtain the porous heating element.

8.  The flavor inhaler cartridge according to claim 7, wherein the carbon source is a phenolic resin.

9.  A flavor inhaler comprising the flavor inhaler cartridge according to any one of claims 1 to 8.

Fig.1

Fig.2

Fig.3

DEVELOPED STATE (PRIOR TO FOLDING)

7

9        7c        9

7a                                                    7b

7d

72a,72                    71                    72b,72

Fig.4

Fig.5

Fig.6

Fig.7

Fig.8

Fig.9

Fig.10

Fig.11A

Fig.11B

Fig.12A

33 — ⊙ — 31,31a

3H

31a

3H

31c — — 31c

Lv

31d

33 — — 31

32a

33f — — 33f

31b

9B

32 — 9A — 7H

3H

31,31b

32

71 — — 32a

7G

Fig.12B

Fig.13

Fig.14

## INTERNATIONAL SEARCH REPORT

International application No.

PCT/JP2016/069012

### A. CLASSIFICATION OF SUBJECT MATTER
*A24F47/00*(2006.01)i, *A61M15/06*(2006.01)i

According to International Patent Classification (IPC) or to both national classification and IPC

### B. FIELDS SEARCHED

Minimum documentation searched (classification system followed by classification symbols)
A24F47/00, A61M15/06

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2014-506486 A (ResMed Ltd.),<br>17 March 2014 (17.03.2014),<br>claims; paragraphs [0013] to [0145]<br>& US 2013/0284169 A1<br>claims; paragraphs [0011] to [0161]<br>& WO 2012/100291 A1      & EP 2667919 A<br>& CN 103328031 A | 1-9 |
| X | JP 2012-506263 A (Buchberger, Helmut),<br>15 March 2012 (15.03.2012),<br>claims; paragraphs [0017] to [0099]<br>& JP 2015-13192 A      & US 2011/0226236 A1<br>claims; paragraphs [0021] to [0128]<br>& US 2014/0283825 A1      & US 2014/0299125 A1<br>& WO 2010/045671 A1      & WO 2010/045670 A1<br>& EP 2358223 A      & EP 2358418 A | 1-9 |

| ☒ | Further documents are listed in the continuation of Box C. | ☐ | See patent family annex. |
|---|---|---|---|

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>01 September 2016 (01.09.16) | Date of mailing of the international search report<br>13 September 2016 (13.09.16) |
|---|---|
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

| International application No. |
| --- |
| PCT/JP2016/069012 |

C (Continuation). DOCUMENTS CONSIDERED TO BE RELEVANT

| Category* | Citation of document, with indication, where appropriate, of the relevant passages | Relevant to claim No. |
| --- | --- | --- |
| X | JP 2014-525237 A (R. J. Reynolds Tobacco Co.), 29 September 2014 (29.09.2014), paragraph [0074] & US 2013/0037041 A1 paragraph [0089] & US 2015/0272225 A1 & WO 2013/022936 A1 & EP 2741626 A & EP 3020291 A | 1-9 |
| X | JP 2015-506182 A (Altria Client Services Inc.), 02 March 2015 (02.03.2015), paragraph [0023] & US 2013/0192623 A1 paragraph [0048] | 1-9 |
| A | JP 2015-524257 A (Nicoventures Holdings Ltd.), 24 August 2015 (24.08.2015), entire text; all drawings & US 2015/0196058 A1 & GB 2504075 A & WO 2014/012905 A1 | 1-9 |
| A | JP 2014-501131 A (Koninklijke Philips N.V.), 20 January 2014 (20.01.2014), entire text; all drawings & US 2013/0263845 A1 & WO 2012/080923 A1 & EP 2651482 A | 1-9 |
| A | JP 2008-545943 A (Vapore, Inc.), 18 December 2008 (18.12.2008), entire text; all drawings & US 2009/0220222 A1 & US 2011/0210458 A1 & WO 2006/124757 A2 | 1-9 |
| A | JP 2012-149282 A (Sumitomo Electric Toyama Co., Ltd.), 09 August 2012 (09.08.2012), entire text; all drawings & US 2013/0295459 A1 & WO 2012/098941 A1 & EP 2666890 A1 & CN 103328693 A | 1-9 |
| A | JP 8-232003 A (Sumitomo Electric Industries, Ltd.), 10 September 1996 (10.09.1996), entire text; all drawings & US 5672387 A & US 5747112 A & EP 696649 A1 | 1-9 |
| A | JP 2003-119085 A (National Institute of Advanced Industrial Science and Technology), 23 April 2003 (23.04.2003), entire text; all drawings & JP 2005-272300 A & US 2005/0020431 A1 & US 2007/0032371 A1 & WO 2003/014042 A1 & EP 1449819 A1 & KR 10-2004-0032889 A | 1-9 |

Form PCT/ISA/210 (continuation of second sheet) (January 2015)

**REFERENCES CITED IN THE DESCRIPTION**

**Patent documents cited in the description**

- JP 5612585 B
- JP 2012149282 A
- JP H08232003 B
- JP 2003119085 A